Europäisches Patentamt

European Patent Office

Office européen des brevets

(11) Publication number: **0 241 210 B1**

(19)
(11)

## EUROPEAN PATENT SPECIFICATION

(12)

(45) Date of publication of patent specification: **08.07.92**  (51) Int. Cl.⁵: **C12N 15/58**, C12N 9/64, A61K 37/54

(21) Application number: **87302837.7**

(22) Date of filing: **01.04.87**

(54) **Modified enzyme.**

(30) Priority: **02.04.86 GB 8608020**

(43) Date of publication of application: **14.10.87 Bulletin 87/42**

(45) Publication of the grant of the patent: **08.07.92 Bulletin 92/28**

(84) Designated Contracting States: **BE CH DE ES FR GB GR IT LI LU NL SE**

(56) References cited:
EP-A- 0 093 619
WO-A-86/01538

PROCEEDINGS OF THE NATIONAL ACADEMY OF SCIENCES OF THE USA, vol. 81, no. 17, September 1984, pages 5355-5359, Washington, US; T. NY et al.: "The structure of the human tissue-type plasminogen activator gene: Correlation of intron and exon structures to functional and structural domains"

(73) Proprietor: **BEECHAM GROUP PLC Beecham House Great West Road Brentford Middlesex TW8 9BD(GB)**

(72) Inventor: **Browne, Michael Joseph Beecham Pharmaceuticals Great Burgh Yew Tree Bottom Road Epsom Surrey KT18 5XO(GB)**
Inventor: **Robinson, Jeffery Hugh Beecham Pharmaceuticals Great Burgh Yew Tree Bottom Road Epsom Surrey KT18 5XO(GB)**

(74) Representative: **Valentine, Jill Barbara et al Beecham Pharmaceuticals Patents & Trade Marks Dept. Great Burgh Yew Tree Bottom Road Epsom Surrey KT18 5XO(GB)**

FEBS LETTERS, vol. 163, no. 1, October 1983, pages 37-41, Elsevier Science Publishers B.V., Amsterdam, NL; L. BANYAI et al.: "Common evolutionary origin of the fibrin-binding structures of fibronectin and tissue-type plasminogen activator"

PROCEEDINGS OF THE NATIONAL ACADEMY OF SCIENCES OF THE USA, vol. 83, no. 13, July 1986, pages 4670-4674, Washington, US; A.J. VAN ZONNEVELD et al.: "Autonomous functions of structural domains on human tissue-type plasminogen activator"

## Description

The present invention relates to modified fibrinolytic enzymes, in particular modified plasminogen activators, their preparation, pharmaceutical compositions containing them and their use in the treatment of thrombotic disease.

Plasminogen activators are a class of proteases that convert plasminogen into plasmin. Human plasminogen activators may be grouped into two classes based on immunological criteria, molecular weight and polypeptide composition (see Collen, D. et al, 1982, Thromb. Haemostas., 48, 294-296). One major type, the urokinase-type plasminogen activators (u-PA) resemble the urinary enzyme urokinase, whereas the other major type, tissue-type plasminogen activators (t-PA), resemble extrinsic activators found in blood, activators extracted from tissues by chaotropic salts and activators secreted by cultured melanoma cells (see Rijken, D.C. and Collen, D., 1981, J. Biol. Chem., 256, 7035-7041; Rijken, D.C. et al, J. Lab. Clin. Med., 97, 477-478).

The term tissue-type or urokinase-type plasminogen activator denotes a plasminogen activator of the group having the immunological properties defined for t-PA or u-PA at the XXVIII Meeting of the International Committee on Thrombosis and Haemostasis, Bergamo, Italy, 27 July 1982.

The term plasminogen activators as used herein includes the human plasminogen activators discussed above, such as t-PA, urokinase, pro-urokinase, as well as t-PA or urokinase muteins (mutated proteins) such as those disclosed in EP-A-O 201 153 or EP-A-O 207 589.

The sequence of amino acids making up the enzymes t-PA and urokinase and the nucleotide sequence for the cDNA which codes for the enzymes are known (see Pennica et al, 1983; Nature, 301, 214 and EP-0092182-A).

Native t-PA is composed of a B or light (L) and an A or heavy (H) chain. The B chain contains the active site of the enzyme.

It has been shown (Ny, T. et al, 1984; Proc. Natl. Acad. Sci. U.S.A., 81, 5355) that the A chain exhibits a number of structural and functional domains which are homologous to structures found in other plasma proteins.

Fibronectin has twelve finger-domains per monomer, responsible for fibrin-affinity (Eur. J. Biochem. 154, 15-29 (1986)). The amino acid sequences of these finger domains are known (EMBO J.4, 1755 - 1759 (1985); Eur. J. Biochem. 128, 605-623 (1982); Proc. Natl. Acad. Sci. USA 80, 137-141 (1983)). It has been shown (J. Biol. Chem. 260, 5328-5341 and 13666-13676 (1985)) that part of Factor XII shows structural homology with the finger-domains of fibronectin. It has also been shown (Bányai, L. et al, 1983; FEBS Lett., 163, 37) that a part of the t-PA enzyme shows structural homology with the finger-domains of fibronectin. This region from amino acid residue 6 to 43 has been termed the t-PA finger domain. The genetic information for this domain lies on a single exon (Ny, T. et al, 1984; Proc. Natl. Acad. Sci. U.S.A., 81, 5355). The term "finger domain" will be used hereinafter to refer to an amino acid sequence showing structural homology with the finger-domains of fibronectin, or the sequence of a fibronectin finger-domain itself.

Another part of the t-PA enzyme shows structural homology with human and murine epidermal growth factors. This region from amino acid residues 44 to 91 has been termed the "growth factor domain" (Banyai, L. et al, 1983; FEBS lett. 163, 37). The genetic information which codes for the major part of this domain, residues 51 to 86 inclusive, and partially codes for residues 50 and 87, lies on a single exon (Ny, T. et al, 1984; Proc. Nat. Acad. Sci. U.S.A., 81, 5355). The region from the first to last cysteine residue within this region, residues 51 and 84, defines a triple-disulphide-linked structure which will be referred to herein as the growth-factor domain. The t-PA A-chain also contains two triple disulphide-bonded structures or kringle domains. Residues 92 to 173 inclusive will be referred to herein as the kringle 1 ($K_1$) domain. Residues 180 to 261 will be referred to herein as the kringle 2 ($K_2$) domain.

The applicants have now identified modified forms of plasminogen activators which retain fibrinolytic activity.

According to the present invention there is provided a fibrinolytically active plasminogen activator which has been modified by the addition of one or more finger domains.

In a preferred aspect, the or each finger domain is derived from native t-PA.

The amino acid sequence of various native forms of t-PA are known. The abovementioned Nature 1983 reference discloses the sequence for the L-chain and the mature S-chain forms of t-PA, also discussed by Vehar et.al., Biotechnology, 1984, 2, 1051-7 in which the processing of initially formed t-PA by removal of a pro-sequence to give the S-chain form is reported. Pohl et.al., FEBS letters, 1984, Vol. 168 No.1, 29-32, refers to the N-terminal multiplicity of t-PA and discloses the U-chain form. The numbering system for the amino acid sequence of t-PA used herein is that described in the Nature 1983 reference for mature (S-chain) t-PA in which the N-terminal serine is numbered 1. By this system, L-chain t-PA has an N-terminal glycine residue at position -3 and U-chain t-PA has an N-terminal valine at position 4.

Where a finger domain is derived from t-PA, the finger domain sequence may optionally extend at the N-terminus to include residues proceding residue 6 of native t-PA, such as residues 4 and 5, 1 to 5 or -3 to 5 respectively of the U-, S- or L-chain forms of native t-PA. The finger domain sequence preferably extends at the N-terminus to comprise residues 1 to 5 of native t-PA.

Preferably the modification is at the N-terminus of the plasminogen activator.

Each finger domain sequence may optionally be linked to a second sequence of amino acids which corresponds to the growth-factor domain of native t-PA. Thus, representing the finger domain sequence (for example residues 6 to 43 of t-PA) as F and the growth-factor domain sequence (residues 51 to 84 of t-PA) as G, the modified plasminogen activator (modified PA) of the invention comprises one or more N-terminal units of the form A-F-X- and/or A-F-Y-G-Z-, where X, Y and Z represent bonds or linking sequences of amino acids which may be introduced synthetically during the preparation of the modified PA and/or be derived from native t-PA sequences adjacent the F and G domains and A is an optional N-terminal extension of the F domain.

In a preferred aspect, the linking sequence X comprises amino acid residues selected from the residues 44 to 50 and 85 to 91, and more preferably comprises residues 44 to 50 and 88 to 91, of native t-PA, optionally linked at the C-terminus to a sequence of amino acids, such as -pro-gly-. The linking sequence Y preferably comprises residues selected from the residues 44 to 50 of native t-PA, and more preferably comprises residues 44 to 50. The linking sequence Z preferably comprises residues selected from the residues 85 to 91 of native t-PA and more preferably comprises residues 85 to 91, optionally linked at the C-terminus to a sequence of amino acids such as -pro-gly-.

It will be appreciated that to prevent cleavage of the additional domain(s) from one another or from the modified plasminogen activator in vivo, inter-domain linking sequences and linking sequences between the additional domain(s) and the plasminogen activator molecule should be chosen so as to avoid the presence of a site susceptible to proteolytic cleavage. Thus, in particular, the linking sequence X or Z will preferably end with a residue other than arginine or lysine.

The modified plasminogen activator of the invention preferably comprises up to 6 additional finger domains, more preferably up to 2, most preferably 1, each of which may optionally be linked to a growth factor domain.

The plasminogen activator modified in accordance with the present invention is preferably t-PA or a t-PA mutein.

It is understood that the tissue-type plasminogen activator modified in accordance with the present invention encompasses all variant forms of native t-PA such as described above.

t-PA muteins which may be modified in accordance with the present invention include:

(i) t-PA modified in the region of the growth factor domain as disclosed in EP-A-O 207 589; and

(ii) t-PA comprising an amino acid without a charged side chain in place of lysine at position 277, as disclosed in EP-A-O 201 153.

The modified plasminogen activator of the invention may be derivatised to provide pharmaceutically useful conjugates analogous to known plasminogen activator-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b) an enzyme-protein conjugate as disclosed in EP-A-O 152 736, comprising at least one optionally blocked fibrinolytic enzyme linked by way of a site other than the catalytic site responsible for fibrinolytic activity to at least one human protein;

(c) a protein-polymer conjugate as disclosed in EP-A-O 183 503 comprising a pharmaceutically useful protein linked to at least one water soluble polymer by means of a reversible linking group; or

(d) an enzyme conjugate as disclosed in EP-A-O 184 363 comprising a plurality of fibrinolytic enzymes linked together through the active centres thereof by means of a removable blocking group.

The modified plasminogen activator of the invention may take the place of native plasminogen activator as the enzyme or (human) protein component, as appropriate, of any of the conjugates described above.

The modified plasminogen activator of the invention or conjugate thereof can be further derivatised such that any catalytic site essential for fibrinolytic activity is optionally blocked by a removable blocking group.

The above mentioned derivatives of the modified plasminogen activator may be used in any of the methods and compositions described hereinafter for the modified plasminogen activator itself.

As used herein the expression 'removable blocking group' includes groups which are removable by hydrolysis at a rate such that the pseudo-first order rate constant for hydrolysis is in the range of $10^{-6}$ sec$^{-1}$ to $10^{-2}$ sec$^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$ alkylphenyl.

In a further aspect, the invention provides a process for preparing modified plasminogen activator according to the invention which process comprises expressing DNA encoding said modified plasminogen activator in a recombinant host cell and recovering the modified plasminogen activator product.

The modification of the plasminogen activator can therefore be carried out by conventional genetic engineering techniques in which the cDNA which codes for the plasminogen activator is modified and the modified cDNA expressed in a prokaryote or eukaryote host, preferably a eukaryote host.

The DNA polymer comprising a nucleotide sequence that encodes the modified plasminogen activator also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

i) preparing a replicable expression vector capable, in a host cell, of expressing a DNA polymer comprising a nucleotide sequence that encodes said modified plasminogen activator;

ii) transforming a host cell with said vector;

iii)culturing said transformed host cell under conditions permitting expression of said DNA polymer to produce said modified plasminogen activator; and

iv) recovering said modified plasminogen activator.

The invention also provides a process for preparing the DNA polymer by the condensation of appropriate mono-, di- or oligomeric nucleotide units.

The preparation may be carried out chemically, enzymatically, or by a combination of the two methods, in vitro or in vivo as appropriate. Thus, the DNA polymer may be prepared by the enzymatic ligation of appropriate DNA fragments, by conventional methods such as those described by D. M. Roberts et al in Biochemistry 1985, 24, 5090-5098.

The DNA fragments may be obtained by digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70°C, generally in a volume of 50µl or less with 0.1-10µg DNA.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 50µl or less.

Enzymatic ligation of DNA fragments may be carried out using a DNA ligase such as T4 DNA ligase in an appropriate buffer at a temperature of 4°C to ambient, generally in a volume of 50µl or less.

The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

Preferably the DNA polymer is prepared by ligating a first DNA molecule comprising a DNA sequence encoding the finger domain amino acid sequence with a second DNA molecule comprising a DNA sequence encoding the plasminogen activator to be modified.

The first DNA molecule may optionally comprise a DNA sequence encoding the growth-factor domain amino acid sequence or the molecule may be provided in fragments which together encode both domains.

Where more than one finger domain addition is required, this may be achieved by the successive ligation of further DNA molecules encoding the finger domain and optionally the growth-factor domain to the DNA encoding the plasmogen activator. Alternatively, the first DNA molecule may encode the required number of domains and be added to the plasminogen activator DNA in a single step. A combination of these two methods may also be employed.

The DNA molecules may be obtained by digestion with suitable restriction ezymes of vectors carrying the required coding sequences.

The first DNA molecule may be obtained by digestion of the cDNA which encodes a finger domain, such as t-PA or a t-PA mutein containing the native finger domain, and optionally the growth-factor domain, coding sequence with suitable restriction enzymes to provide a DNA fragment including the sequence encoding the required domain(s).

The ligation of the first and second DNA molecules may be achieved directly or by means of a linker. The ligation may be designed to avoid the presence of an enzymatic cleavage site such as -arg/lys-Q- where Q is any naturally occurring L-amino acid, as discussed above.

In the preferred embodiment, a DNA molecule comprising the sequence encoding the finger domain of t-PA may be obtained by the digestion with the restriction enzymes HindIII and MaeII of the plasmid pTRE24 disclosed in EP-A-O 207 589. This plasmid comprises the DNA sequence encoding t-PA but with the portion encoding amino acid residues 51 to 87 deleted. The digestion provides a DNA fragment including portions encoding residues 1 to 50 and 88 to 90, and partially encoding residue 91:

```
                              MaeII site

                                   |

           5' AGT ACC AGG GCC A    3'coding strand

           3' TCA TGG TCC CGG TGC 5'

                                   |



               ser thr arg ala thr

               50  88  89  90  91
```

This fragment may be blunt-ended and one or more linkers added, such as the BamHI linker from Amersham (DCl203), the linker digested to provide a sticky end such as:

```
               5' CCC G        3'

               3' GGG CCT AG 5'
```

which can be ligated with the BglII site adjacent the serine 1 codon of t-PA:

```
                   BglII site

                      |

           5' GA TCT TAC 3'coding strand

           3'       A ATG 5'

                      |

                   ser tyr

                    1    2
```

The resulting sequence at the ligation site is as follows:

```
5' CCC GGA TCT TAC 3'
3' GGG CCT AGA ATG 5'
```

```
                pro gly ser tyr
                         1    2
```

from which it can be seen that there is no trypsin-like cleavage site immediately N-terminal to the serine 1.

A DNA molecule comprising the sequence encoding both the finger domain and the growth-factor domain of t-PA may similarly be obtained by the digestion of native t-PA cDNA with enzymes HindIII and MaeII to yield a DNA fragment including a portion encoding residues 1 to 90 and partially encoding residue 91. This fragment may be subsequently treated as for the finger domain fragment described above.

The expression of the DNA polymer encoding the modified plasminogen activator in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and ligating said linear segment and one or more DNA molecules which, together with said linear segment, encode the modified plasminogen activator.

The ligation of the linear segment and more than one DNA molecule may be carried out simultaneously or sequentially as desired.

Thus, the DNA polymer may be preformed or formed during the construction of the vector, as desired.

In particular, the replicable expression vector may be prepared by cleaving a vector compatible with the host cell to provide a linear DNA segment having an intact replicon, and ligating said linear segment with a first DNA molecule comprising a DNA sequence encoding the finger domain amino acid sequence and a second DNA molecule comprising a DNA sequence encoding the plasminogen activator to be modified.

The choice of vector will be determined in part by the host cell, which may be prokaryotic or eukaryotic, preferably eukaryotic. Suitable vectors include plasmids, bacteriophages, recombinant viruses and cosmids.

The preparation of the replicable expression vector may be carried out conventionally with appropriate enzymes for restriction, polymerisation and ligation of the DNA, by procedures described in, for example, Maniatis et al cited above. Restriction, polymerisation and ligation may be performed as described above for the preparation of the DNA polymer.

The recombinant host cell is prepared, in accordance with the invention, by transforming a host cell with a replicable expression vector of the invention under transforming conditions. Suitable transforming conditions are conventional and are described in, for example, Maniatis et al cited above, or "DNA Cloning", D.M. Glover ed., IRL Press Ltd, 1985.

The choice of transforming conditions is determined by the host cell. Thus, a bacterial host such as E. coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]-propane-sulphonic acid, RbCl and glycerol. Mammalian cells in culture may be transformed by calcium co-precipitation of the vector DNA onto the cells.

The invention also extends to a host cell transformed with a replicable expression vector of the invention.

Culturing the transformed host cell under conditions permitting expression of the DNA polymer is carried out conventionally, as described in, for example, Maniatis et al and "DNA Cloning" cited above. Thus, preferably the cell is supplied with nutrient and cultured at a temperature below 45°C.

The modified plasminogen activator expression product is recovered by conventional methods according to the host cell. Thus, where the host cell is bacterial, such as E.coli it may be lysed physically, chemically or enzymatically and the protein product isolated from the resulting lysate. Where the host cell is mammalian, the product may generally be isolated from the nutrient medium.

The DNA polymer may be assembled into vectors designed for isolation of stable transformed mammalian cell lines expressing the modified t-PA; e.g. bovine papillomavirus vectors (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al.,European Patent Application No. 0093619, 1983).

It will be appreciated that, depending upon the host cell, the modified plasminogen activator prepared in accordance with the invention may be glycosylated to varying degrees. As observed by Pohl et.al., Biochemistry, 1984, 23, 3701-3707, varying degrees of glycosylation may be found in unmodified, naturally occurring t-PA. The modified t-PA of the invention is understood to include such glycosylated variations.

In the modified plasminogen activator of the invention, any plasminogen activator chain which has been modified by the addition of one or more finger domains may be employed as one chain of a fibrinolytically active hybrid protein such as disclosed in EP-O155387. The modified chain, DNA encoding the modified chain and a hybrid protein comprising the modified chain linked to a chain of another protease, such that the hybrid protein has a catalytic site essential for fibrinolytic activity which is optionally blocked by a removable blocking group, all form part of the invention. The modified plasminogen activator chain may be prepared by separation from the other chain of the plasminogen activator by mild reduction. Alternatively the modified chain may be prepared by expressing DNA encoding therefor in a recombinant host cell and recovering the modified chain product. The hybrid protein comprising the modified plasminogen activator chain linked to a chain of another protease may be prepared by (a) mixing said chains under oxidative conditions; or (b) ligating DNA encoding said chains and expressing the ligated DNA in a prokaryote or eukaryote host; and thereafter optionally blocking the catalytic site of the hybrid protein with a removable blocking group. The oxidation and reduction conditions are as generally described in EP-0155387. The hybrid protein may be used in any of the methods and compositions described hereinafter for the modified plasminogen activator (PA) itself.

The modified PA of the invention is suitably administered in the form of a pharmaceutical composition.

Accordingly the present invention also provides a pharmaceutical composition comprising modified PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the modified PA in solution and a local anaesthetic such as lignocaine to ease pain at the site of injection. Generally, the modified PA will be supplied in unit dosage form for example as a dry powder or water free concentrate in a hermetically sealed container such as an ampoule or sachette indicating the quantity of protein in activity units. Where the modified PA includes a removable blocking group an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration. The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a mature, fresh or nascent thrombus will generally receive a daily dose of from 0.01 to 10 mg/kg of body weight either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of modified PA of the invention.

The invention also provides a modified PA of the invention for use as an active therapeutic substance and, in particular, for use in the treatment of thrombotic diseases.

The following Examples illustrate the invention.

Methods used for Examples

DNA cleavage

In general the cleavage of about 1μg of plasmid DNA or DNA fragments was effected using about 5 units of a restriction enzyme (or enzymes) in about 20μl of an appropriate buffer solution.

Generation of blunt ends: If blunt ends were required they were produced by treating the DNA preparation with DNA Polymerase I, Klenow fragment (Molecular Cloning, A Laboratory Manual, Cold Spring Harbor Laboratory 1982).

Ligation of DNA Fragments: Ligation reactions were carried out as described (Maniatis et al Molecular Cloning - A Laboratory Manual, Cold Spring Harbor Laboratory 1982).

Transformation of plasmid DNA into E. coli HB101 cells was as described by Hanahan (DNA Cloning Vol I Chapter 6, D.M. Glover ed. IRL Press, 1985) in Protocol 3 except that incubation with RFI was for 5 minutes.

Plasmid preparation: Large scale preparation of plasmid DNA and plasmid mini-preparations were carried out as described in Maniatis et al (Molecular Cloning - A Laboratory Manual, Cold Spring Harbor Laboratory, 1982).

Isolation of DNA fragments from low-melting-point (LMP) agarose gels

DNA fragments were isolated from LMP agarose gels as described by Maniatis et al (Molecular Cloning, A Laboratory Manual, 1982, Cold Spring Harbor).

Ligation of Synthetic Linkers to DNA

Synthetic linkers encoding restriction enzyme sites, were kinased and ligated to blunt-ended DNA as described by Maniatis et al (Molecular Cloning, A Laboratory Manual, 1982, Cold Spring Harbor).

Fibrin/agar overlay for detection of t-PA expression

Cell preparation: cells were trypsinised and plated out at $10^6$ cells per 60mm dish and left overnight in growth medium (10% Serum, 1% stock solution of penicillin/streptomycin, 1% stock Glutamine, 1% stock solution of non-essential amino acids; Flow Laboratories, in Eagles MEM) at 37°C in a humidified incubator in an atmosphere of 5% $CO_2$/95% air.

Transfection procedure: The transfection used (calcium coprecipitation) is described in 'DNA Cloning' Ed. D.M. Glover (Chap. 15, C. Gorman). Glycerol shock and 10mM butyrate treatment were used.

Overlay: Agarose (Indubiose $A_{37}$), 2.4g in 95ml Eagles MEM (Gibco) heated to melting point in a bunsen flame, was then placed in a water bath maintained at 48°C. 5.6 ml of fibrinogen (20mg/ml) were diluted 1:1 with 5.6 ml of Eagles MEM (containing an extra 7 mg/ml of NaCl) and retained on ice. 3.3 ml of Eagles MEM (no additions) were aliquoted into a bijou containing 86$\mu$l of bovine thrombin at 50 NIH Units/ml (retained on ice). The cells were washed 3 times with Eagles MEM to remove serum. The thrombin and fibrinogen were warmed to 37°C in a water bath. 9.5 ml agarose were aliquoted into a pre-warmed universal. The thrombin was added to the agar, followed by the fibrinogen. The gel was poured over the cell layer and incubated at 37°C until lysis zones appeared.

Example 1

Construction of t-PA mutein cDNAs

1. Preparation of DNA encoding the t-PA finger and growth factor domains

6 $\mu$g of the plasmid pTRE15 (described in EP-A-O 201 153) was digested with 19.4 units of the enzyme MaeII (Boehringer) for 2$\frac{1}{2}$h at 50°C in a volume of 75$\mu$l using the buffer described by the manufacturer. The MaeII cut DNA was flush-ended using DNA polymerase I Klenow fragment. Kinased BamHI linkers (0.1 $A_{260}$ units; Amersham DCl203) of sequence 5′CCCGGATCCGGG3′ were ligated to 5$\mu$g of the flush-ended MaeII fragments. After ligation excess linkers were removed and cohesive "sticky-ends" created by digestion with BamHI. The BamHI digest was electrophoresed on a 1% low melting point agarose gel. On this gel there was a group of three bands with mobility between that of the 1353 and 872 base pair fragments found in a lambda DNA-HindIII/X174RF-HaeIII digest (Drigest TMIII, Pharmacia). The largest of these three bands, approximately 1200bp, was recovered (no special attempt was made to ensure that the other hands of similar mobility did not contaminate this preparation, since undesired molecules are eliminated in the cloning strategy). The 1200bp fragment was digested with HindIII to create the desired approx. 470bp fragment with a HindIII 5′ sticky end and a 3′ BamHI sticky end (converted from the MaeII site).

No attempt was made to separate the DNA fragments resulting from this HindIII digest. This DNA preparation is known as preparation A.

2. Preparation of DNA encoding the t-PA finger domain

The method used follows that in 1. (above) except that the starting point was plasmid pTRE24 (described in EP-A-O 207 589 and that after BamHI digestion only two DNA bands of mobility between the 1353 and 872 base pair markers were visible - the band of highest molecular weight approx. 1100bp (possibly a doublet) was recovered. After HindIII digestion the DNA was known as preparation B. Note that in this preparation the desired HindIII/BamHI fragment (approx. 360bp) is shorter than the one described in 1. (above) due to the absence of the growth factor coding domain.

3. Preparation of recipient (partial) t-PA cDNA molecules

Plasmids pTRE15 (10μg), pTRE18 (5μg) (described in EP-A-O-201 153) and pTRE24 (5μg) were digested with BglII and SstI. The digests were electrophoresed on a 1% low melting point agarose gel; a DNA fragment of approx. 1230bp was recovered from each of the pTRE15 and pTRE18 digests (fragment C and D respectively) and a DNA fragment of approximately 1120bp was recovered from the pTRE24 digest (fragment E). The BglII and SstI sites used to generate these fragments correspond to those found at positions 187 and 1417 in the DNA sequence described by Pennica et al., 1983 (Nature 301, 214). The BglII- SstI fragment from pTRE24 is shortened due to the absence of the growth factor coding domain (residues 51 to 87). These BglII-SstI fragments thus encode the whole mature t-PA (or t-PA mutein) A-chain and part of the t-PA (or t-PA mutein) B-chain, as appropriate to the starting plasmid).

4. Preparation of the expression vector for t-PA muteins

10μg of the plasmid pTRE15 was digested with HindIII and SstI and the approx. 6kb fragment recovered from a 0.8% low melting point agarose gel (fragment F). In addition to vector sequences this fragment also encodes part of the t-PA B-chain and 3′ untranslated region. The SstI site corresponds to that at position 1417 in the sequence described by Pennica et al., (1983).

5. Ligation of DNA molecules and transformation into E. coli HB101

The following ligations were performed in volumes of 10 μl. Quantities of DNA were approximated by visual inspection of fragments on ethidium bromide stained agarose gels.
1. Preparation A 20ng
Fragment C 110ng
Fragment F 150ng
2. Preparation B 20ng
Fragment C 110ng
Fragment F 150ng
3. Preparation A 20ng
Fragment D 60ng
Fragment F 150ng
4. Preparation B 20ng
Fragment D 60ng
Fragment F 150ng
5. Preparation A 20ng
Fragment E 120ng
Fragment F 150ng
6. Preparation B 20ng
Fragment E 120ng
Fragment F 150ng
The ligations were used to transform E. coli strain HB101. Transformants were selected by growth in the presence of ampicillin (100μg/ml).
Restriction enzyme analysis (BglII plus SstI digestion) of the DNA from mini- preparations was used to identify constructs of the desired type, molecules consistent with the following coding potential were obtained (structures are abbreviated to indicate domains only):

| Ligation | | | | | | | Plasmid name |
|---|---|---|---|---|---|---|---|
| 1 | F G F G | $K_1$ | $K_2$ | B-chain | | | pTRE60 |
| 2 | F F G | $K_1$ | $K_2$ | B-chain | | | pTRE61 |
| 3 | F G F G | $K_1$ | $K_2$ | modified B-chain:ile277 | | | pTRE62 |
| 4 | F F G | $K_1$ | $K_2$ | -ditto- | | | pTRE63 |
| 5 | F G F | $K_1$ | $K_2$ | B-chain | | | pTRE64 |
| 6 | F F | $K_1$ | $K_2$ | B-chain | | | pTRE65 |

Where

F = finger domain (residues 6 to 43)

G = growth factor domain (residues 51 to 84)

$K_1$ = kringle 1 domain

$K_2$ = kringle 2 domain

B-chain = serine protease domain

It is appreciated that joining the extra domains onto the N-terminus of t-PA (or its muteins) by means of fusion of a BamHI linker and the BglII site (corresponding to position 187 in Pennica et al., 1983) ensures that arg-1 is replaced by gly-1, due to a change in the codon from AGA to GGA (the 5′ G of the GGA codon being donated by the BamHI linker). The change was effected to remove an inter-domain site potentially susceptible to proteolytic cleavage.

Where removal of excess BamHI linkers from DNA preparations A or B (example 1) is incomplete, there exists the possibility of retention of one (or more) complete BamHI linkers immediately 5′ to the BamHI/BglII junction. With each complete BamHI linker, four extra amino acids coded by the linker (proline, glycine, serine, glycine) are accordingly added to the mutant protein. BamHI digestion of plasmids pTRE60 to 65 revealed that only pTRE63 apparently had one extra cleavage site, compatible with the presence of one or more complete BamHI linker(s).

Example 2

Expression of the mutant t-PA cDNA molecules

Construction of the mutant cDNAs using the vector moiety of pTRE15 allowed direct examination of the coding potential of the new genes without further manipulation at the DNA level. Plasmid preparations pTRE60 to 65 inclusive (ultimately derived from ligations 1 to 6) were used in the transient expression assay described in EP-A-O 207 589 except that mouse L929 cells were substituted for C127 cells. Overlay of the cultures with a "fibrin/agarose" gel revealed that all six mutant cDNA molecules coded for proteins with plasminogen activator activity.

The structures of the t-PA muteins encoded by the mutant cDNA molecules are believed to be as follows:

[GARSYQVI]F[HSVPVKS]G[EIDTRATPG]-t-PA

[GARSYQVI]F[HSVPVKSTRATPG]-t-PA.

[GARSYQVI]F[HSVPVKS]G[EIDTRATPG]-t-PA($lys_{277}$->$ile_{277}$)

[GARSYQVI]F[HSVPVKSTRAT(PGSG)$_n$PG]-t-PA ($lys_{277}$->$ile_{277}$)

[GARSYQVI]F[HSVPVKS]G[EIDTRATPG]-t-PA(des $cys_{51}$ to $asp_{87}$)

[GARSYQVI]F[HSVPVKSTRATPG]-t-PA(des $cys_{51}$ to $asp_{87}$)

where t-PA represents native t-PA with an N-terminal serine residue, F represents residues 6 to 43 of native t-PA, G represents residues 51 to 84 of native t-PA, n is an integer of at least one, and the symbols in square brackets represent amino acid residues according to the single letter amino acid notation.

It will be appreciated that the N-terminal processing of the expressed muteins will vary with the cell line and the expression conditions, by analogy with the processing of native t-PA discussed hereinbefore. The N-terminal variants of the muteins, i.e. where the sequence preceding F is [SYQVI] or [VI] may also be obtainable by the expression of the mutant cDNA molecules.

Example 3

Zymographic analysis of mutant t-PA molecules

Cell preparation: Hela cells were trypsinised and plated out at a density of $1.4 \times 10^4$ cells per cm$^2$ in growth medium (10% Serum (021-6010), 1% stock solution of penicillin/streptomycin (043-5070), 2% sodium bicarbonate solution (043-5080), 1% stock Glutamine (043-5030), in Hepes buffered RPMI 1640 medium (041-2400); Gibco, Paisley, Scotland) at 37°C in a humidified incubator in an atmosphere of 5% $CO_2$/95% air. After 72h the cells were refed and were used for DNA transfection 24h later.

Transfection procedure: Transfection of plasmids pTRE60 and 61 (in Eagles MEM) used calcium coprecipitation as described in 'DNA Cloning' Ed. D.M. Glover (Chap. 15, C. Gorman). Glycerol shock and 5mM butyrate treatment were used. Plasminogen activator secreted by transfected cells was harvested in RPMI 1640 medium (as above but serum-free, containing 4% soybean peptone (Sigma)) for 24h.

Zymography: Modified t-PA from transfected human HeLa cells was analysed by zymography as described by Dodd et al., (1986), Thrombosis and Haemostasis 55(1); 94.

The major protein species produced in both cases was larger than t-PA: in the case of pTRE60 the protein had an apparent Mr of approximately 83,000 and in the case of pTRE61 an apparent Mr of approximately 80,000.

**Claims**
**Claims for the following Contracting States : BE, CH, DE, FR, GB, GR, IT, LU, NL, SE**

1. A fibrinolytically active plasminogen activator which has been modified by the addition of one or more finger domains.

2. A modified plasminogen activator according to claim 1, wherein the or each finger domain is derived from native t-PA.

3. A modified plasminogen activator according to claim 2, wherein the amino acid sequence of the or each finger domain extends at the N-terminus to comprise residues 1 to 5 of native t-PA.

4. A modified plasminogen activator according to any preceding claim, wherein at least one of the finger domains is linked to a second sequence of amino acids which corresponds to the growth factor domain of native t-PA.

5. A modified plasminogen activator according to any preceding claim, wherein the modification is at the N-terminus of the plasminogen activator.

6. A modified plasminogen activator according to claim 1, which comprises one or more N-terminal units of the form A-F-X and/or A-F-Y-G-Z- where F represents the residues 6 to 43 of t-PA, G represents the residues 51 to 84 of t-PA, X, Y and Z represent bonds or linking sequences of amino acids which may be introduced synthetically during the preparation of the modified plasminogen activator and/or be derived from native t-PA sequences adjacent the finger and growth factor domains and A is an optional N-terminal extension of the finger domain.

7. A modified plasminogen activator according to claim 6, wherein the linking sequence X or Z ends with a residue other than arginine or lysine.

8. A modified plasminogen activator according to claim 7, wherein X comprises residues 44 to 50 and 88 to 91 of native t-PA linked at the C-terminus to the sequence -pro-gly-, Y comprises residues 44 to 50 of native t-PA, Z comprises residues 85 to 91 of native t-PA linked at the C-terminus to the sequence -pro-gly-and A comprises residues 1 to 5 of native t-PA.

9. A modified plasminogen activator according to any preceding claim, which comprises 1 to 6 additional finger domains.

10. A modified plasminogen activator according to any preceding claim, wherein the plasminogen activator is native t-PA, t-PA modified in the region of the growth factor domain or t-PA comprising an amino acid without a charged side chain in place of lysine at position 277.

11. A modified plasminogen activator selected from

[GARSYQVI]F[HSVPVKS]G[EIDTRATPG]-t-PA

[GARSYQVI]F[HSVPVKSTRATPG]-t-PA

[GARSYQVI]F[HSVPVKS]G[EIDTRATPG]-t-PA($lys_{277}$->$ile_{277}$)

[GARSYQVI]F[HSVPVKSTRAT(PGSG)$_n$PG]-t-PA($lys_{277}$->$ile_{277}$)

[GARSYQVI]F[HSVPVKS]G[EIDTRATPG]-t-PA(des $cys_{51}$ to $asp_{87}$)·

[GARSYQVI]F[HSVPVKSTRATPG]-t-PA(des $cys_{51}$ to $asp_{87}$)

where t-PA represents native t-PA with an N-terminal serine residue, F represents residues 6 to 43 of native t-PA, G represents residues 51 to 84 of native t-PA, n is an integer of at least one, and the symbols in square brackets represent amino acid residues according to the, single letter amino acid notation, or an N-terminal processing variant thereof.

12. A fibrinolytically active enzyme-protein conjugate in which the catalytic site on the enzyme which is responsible for fibrinolytic activity is blocked by a human protein attached thereto by way of a reversible linking group, wherein at least one of the enzyme and human protein is the modified plasminogen activator according to any of claims 1 to 11.

13. A fibrinolytically active enzyme-protein conjugate comprising at least one fibrinolytic enzyme linked by way of a site other than the catalytic site responsible for fibrinolytic activity to at least one human protein, wherein at least one of the enzyme and human protein is the modified plasminogen activator according to any of claims 1 to 11.

14. A conjugate of the modifed plasminogen activator according to any of claims 1 to 11, wherein said modified plasminogen activator is linked to at least one water-soluble polymer by means of a reversible linking group.

15. An enzyme conjugate comprising a plurality of fibrinolytic enzymes linked together through the active centres thereof by means of a removable blocking group, at least one of said enzymes being the modified plasminogen activator according to any one of claims 1 to 11.

16. A derivative of the modified plasminogen activator according to any one of claims 1 to 11 or conjugate according to any one of claims 12 to 14, in which any catalytic site responsible for fibrinolytic activity is blocked by a removable blocking group.

17. A derivative according to claim 16, wherein the removable blocking group is optionally substituted benzoyl or acryloyl.

18. A derivative according to claim 17, wherein said optional substituents for the benzoyl blocking group include halogen, $C_{1-6}$ alkyl, $C_{1-6}$ alkoxy, $C_{1-6}$ alkanoyloxy, $C_{1-6}$ alkanoylamino, amino and guanidino.

19. A DNA polymer comprising a nucleotide sequence that encodes a modified plasminogen activator according to any of claims 1 to 11.

13

20. A replicable expression vector capable, in a host cell, of expressing a DNA polymer according to claim 19.

21. A host cell transformed with the vector according to claim 20.

22. A plasminogen activator chain which has been modified in accordance with any of claims 1 to 11.

23. A DNA polymer comprising a nucleotide sequence that encodes the modified plasminogen activator chain according to claim 22.

24. A hybrid protein comprising the modified chain according to claim 22 linked to a chain of another fibrinolytically active protease, such that the hybrid protein has a catalytic site essential for fibrinolytic activity which is optionally blocked by a removable blocking group.

25. A pharmaceutical composition comprising modified plasminogen activator according to any of claims 1 to 11, a derivative or conjugate according to any of claims 12 to 18 or hybrid protein according to claim 24 in combination with a pharmaceutically acceptable carrier.

26. A modified plasminogen activator according to any of claims 1 to 11, a derivative or conjugate according to any of claims 12 to 18 or hybrid protein according to claim 24 for use as an active therapeutic substance.

27. A modified plasminogen activator according to any of claims 1 to 11, a derivative or conjugate according to any of claims 12 to 18 or hybrid protein according to claim 24 for use in the treatment of thrombotic disease.

28. Use of a modified plasminogen activator according to any of claims 1 to 11, a derivative or conjugate according to any of claims 12 to 18 or hybrid protein according to claim 24 for the preparation of a medicament for the treatment of thrombotic disease.

29. A process for preparing modified plasminogen activator according to claim 1 or a derivative according to claim 16, which process comprises expressing DNA encoding said modified plasminogen activator in a recombinant host cell and recovering the modified plasminogen activator product and thereafter optionally blocking any catalytic site with a removable blocking group.

30. A process for preparing a DNA polymer according to claim 14, by the condensation of appropriate mono-, di-or oligomeric nucleotide units.

31. Modified plasminogen activator obtainable by the process of claim 29.

**Claims for the following Contracting State : ES**

1. A process for preparing a fibrinolytically active plasminogen activator which has been modified by the addition of one or more finger domains and in which any catalytic site responsible for fibrinolytic activity is optionally blocked by a removable blocking group, which process comprises expressing DNA encoding said modified plasminogen activator in a recombinant host cell and recovering the modified plasminogen activator product and thereafter optionally blocking any catalytic site with a removable blocking group.

2. A process according to claim 1, wherein the or each finger domain is derived from native t-PA.

3. A process according to claim 2, wherein the amino acid sequence of the or each finger domain extends at the N-terminus to comprise residues 1 to 5 of native t-PA.

4. A process according to any preceding claim, wherein at least one of the finger domains is linked to a second sequence of amino acids which corresponds to the growth factor domain of native t-PA.

5. A process according to any preceding claim, wherein the modification is at the N-terminus of the

14

plasminogen activator.

6. A process according to claim 1 for preparing a modified plasminogen activator which comprises one or more N-terminal units of the form A-F-X and/or A-F-Y-G-Z- where F represents the residues 6 to 43 of t-PA, G represents the residues 51 to 84 of t-PA, X, Y and Z represent bonds or linking sequences of amino acids which may be introduced synthetically during the preparation of the modified plasminogen activator and/or be derived from native t-PA sequences adjacent the finger and growth factor domains and A is an optional N-terminal extension of the finger domain.

7. A process according to claim 6, wherein the linking sequence X or Z ends with a residue other than arginine or lysine.

8. A process according to claim 7, wherein X comprises residues 44 to 50 and 88 to 91 of native t-PA linked at the C-terminus to the sequence -pro-gly-, Y comprises residues 44 to 50 of native t-PA, Z comprises residues 85 to 91 of native t-PA linked at the C-terminus to the sequence -pro-gly- and A comprises residues 1 to 5 of native t-PA.

9. A process according to any preceding claim for preparing a modified plasminogen activator which comprises 1 to 6 additional finger domains.

10. A process according to any preceding claim, wherein the plasminogen activator is native t-PA, t-PA modified in the region of the growth factor domain or t-PA comprising an amino acid without a charged side chain in place of lysine at position 277.

11. A process according to claim 1 for preparing a modified plasminogen activator selected from

**[GARSYQVI]F[HSVPVKS]G[EIDTRATPG]-t-PA**

**[GARSYQVI]F[HSVPVKSTRATPG]-t-PA**

**[GARSYQVI]F[HSVPVKS]G[EIDTRATPG]-t-PA(lys$_{277}$->ile$_{277}$)**

**[GARSYQVI]F[HSVPVKSTRAT(PGSG)$_n$PG]-t-PA(lys$_{277}$->ile$_{277}$)**

**[GARSYQVI]F[HSVPVKS]G[EIDTRATPG]-t-PA(des cys$_{51}$ to asp$_{87}$)**

**[GARSYQVI]F[HSVPVKSTRATPG]-t-PA(des cys$_{51}$ to asp$_{87}$)**

where t-PA represents native t-PA with an N-terminal serine residue, F represents residues 6 to 43 of native t-PA, G represents residues 51 to 84 of native t-PA, n is an integer of at least one, and the symbols in square brackets represent amino acid residues according to the single letter amino acid notation, or an N-terminal processing variant thereof.

12. A process for preparing a DNA polymer comprising a nucleotide sequence that encodes a modified plasminogen activator as defined in any of claims 1 to 11, by the condensation of appropriate mono-, di- or oligomeric nucleotide units.

13. A process for preparing a replicable expression vector capable, in a host cell, of expressing a DNA polymer as defined in claim 12, which process comprises cleaving a vector compatible with said host cell to provide a linear DNA segment having an intact replicon, and combining said linear segment with one or more DNA molecules which, together with said linear segment, encode the modified plasminogen activator, under ligating conditions.

14. A process for preparing a host cell transformed with the vector defined in claim 13, which process comprises transforming a host cell with said vector under transforming conditions.

15. A process for preparing a plasminogen activator chain which has been modified in accordance with any of claims 1 to 11, which process comprises separating said chain from the other chain thereof by mild reduction, or expressing DNA encoding therefor in a recombinant host cell and recovering the modified chain product.

**16.** A process for preparing hybrid protein comprising the modified chain defined in claim 15 linked to a chain of another fibrinolytically active protease, such that the hybrid protein has a catalytic site essential for fibrinolytic activity which is optionally blocked by a removable blocking group, which process comprises mixing said chains under oxidative conditions or ligating DNA encoding said chains and expressing the ligated DNA in a prokayote or enkayote host, and thereafter optionally blocking the catalytic site of the hybrid protein product with a removable blocking group.

**17.** Use of a modified plasminogen activator as defined in any of claims 1 to 11 for the preparation of a medicament for the treatment of thrombotic disease.

**18.** A fibrinolytically active plasminogen activator obtainable by the process of claim 1.

**Revendications**
**Revendications pour les Etats contractants suivants : BE, CH, DE, FR, GB, GR, IT, LU, NL, SE**

**1.** Activateur de plasminogène actif fibrinolytiquement qui a été modifié par l'addition d'un ou de plusieurs domaines de doigt.

**2.** Activateur de plasminogène modifié suivant la revendication 1, dans lequel le ou chaque domaine de doigt provient de t-PA naturel.

**3.** Activateur de plasminogène modifié suivant la revendication 2, dans lequel la séquence d'acide aminé du ou de chaque domaine de doigt se prolonge au N-terminal pour comprendre les résidus 1 à 5 du t-PA naturel.

**4.** Activateur de plasminogène modifié suivant l'une quelconque des revendications précédentes, dans lequel au moins un des domaines du doigt est lié à une seconde séquence d'acides aminés qui correspond au domaine de facteur de croissance de t-PA naturel.

**5.** Activateur de plasminogène modifié suivant l'une quelconque des revendications précédentes, dans lequel la modification est au N-terminal de l'activateur de plasminogène.

**6.** Activateur de plasminogène modifié suivant la revendication 1, qui comprend une ou plusieurs unités N-terminal de la forme A-F-X et/ou A-F-Y-G-Z dans lesquelles F représente les résidus 6 à 43 de t-PA, G représente les résidus 51 à 84 de t-PA, X, Y et Z représentent des liaisons ou des séquences de liaisons d'acides aminés qui peuvent être introduites par synthèse pendant la préparation de l'activateur de plasminogène modifié et/ou provenir des séquences de t-PA naturel adjacentes au domaine du doigt et du facteur de croissance et A est un prolongement N-terminal facultatif du domaine du doigt.

**7.** Activateur de plasminogène modifié suivant la revendication 6, dans lequel la séquence de liaison X ou Z se termine avec un résidu autre que l'arginine ou la lysine.

**8.** Activateur de plasminogène modifié suivant la revendication 7, dans lequel X comprend les résidus 44 à 50 et 88 à 91 du t-PA naturel reliés au C-terminal à la séquence -pro-gly-, Y comprend les résidus 44 à 50 du t-PA naturel, Z comprend les résidus 85 à 91 du t-PA naturel lié au C-terminal à la séquence -pro-gly- et A comprend les résidus 1 à 5 du t-PA naturel.

**9.** Activateur de plasminogène modifié suivant l'une quelconque des revendications précédentes, qui comprend 1 à 6 domaines de doigt supplémentaires.

**10.** Activateur de plasminogène modifié suivant l'une quelconque des revendications précédentes, dans lequel l'activateur de plasminogène est le t-PA naturel, le t-PA modifié dans la région du domaine du facteur de croissance ou le t-PA comprenant un acide aminé sans chaîne latérale chargée à la place de la lysine en position 277.

**11.** Activateur de plasminogène modifié choisi parmi les suivants :

16

$[GARSYQVI]F[HSVPVKS]G[EIDTRATPG]$-t-PA

$[GARSYQVI]F[HSVPVKSTRATPG]$-t-PA

$[GARSYQVI]F[HSVPVKS]G[EIDTRATPG]$-t-PA$(lys_{277}->ile_{277})$

$[GARSYQVI]F[HSVPVKSTRAT(PGSG)_nPG]$-t-PA$(lys_{277}->ile_{277})$

$[GARSYQVI]F[HSVPVKS]G[EIDTRATPG]$-t-PA$(des\ cys_{51}\ à\ asp_{87})$

$[GARSYQVI]F[HSVPVKSTRATPH]$-t-PA$(des\ cys_{51}\ à\ asp_{87})$

dans lesquels t-PA représente le t-PA naturel avec un résidu sérine N-terminal, F représente les résidus 6 à 43 du t-PA naturel, G représente les résidus 51 à 84 du t-PA naturel, n est un entier d'au moins 1 et les symboles entre crochets représentent les résidus d'acide aminé selon la notation des acides aminés par une lettre unique, ou une variante issue du traitement N-terminal de ceux-ci.

12. Conjugué enzyme-protéine actif fibrinolytiquement dans lequel le site catalytique sur l'enzyme qui est responsable de l'activité fibrinolytique est bloqué par une protéine humaine attachée à celle-ci au moyen d'un groupe de liaison réversible, dans lequel l'une au moins de l'enzyme et de la protéine humaine est l'activateur de plasminogène modifié suivant l'une quelconque des revendications 1 à 11.

13. Conjugué d'enzyme-protéine actif fibrinolytiquement comprenant au moins une enzyme fibrinolytique liée au moyen d'un site autre que le site catalytique responsable de l'activité fibrinolytique à au moins une protéine humaine, dans l'une au moins de l'enzyme et de la protéine humaine est l'activateur de plasminogène modifié suivant l'une quelconque des revendications 1 à 11.

14. Conjugué de l'activateur de plasminogène modifié suivant l'une quelconque des revendications 1 à 11, dans lequel cet activateur de plasminogène modifié est lié à au moins un polymère soluble dans l'eau au moyen d'un groupe de liaison réversible.

15. Conjugué d'enzyme comprenant un grand nombre d'enzymes fibrinolytiques reliées ensemble par l'intermédiaire de leurs centres actifs au moyen d'un groupe bloquant pouvant être éliminé, l'une au moins de ces enzymes étant l'activateur de plasminogène modifié suivant l'une quelconque des revendications 1 à 11.

16. Dérivé de l'activateur de plasminogène modifié suivant l'une quelconque des revendications 1 à 11 ou conjugué suivant l'une quelconque des revendications 12 à 14, dans lequel un quelconque site catalytique responsable de l'activité fibrinolytique est bloqué par un groupe bloquant pouvant être éliminé.

17. Dérivé suivant la revendication 16, dans lequel le groupe bloquant pouvant être éliminé est un groupe benzoyle ou acryloyle éventuellement substitué.

18. Dérivé suivant la revendication 17, dans lequel ces substituants éventuels pour le groupe bloquant benzoyle, comprennent un atome d'halogène, des groupes alkyle en $C_{1-6}$ , alcoxy en $C_{1-6}$ , alcanoyloxy en $C_{1-6}$ , alkanoylamino en $C_{1-6}$ , amino ou guanidino.

19. Polymère d'ADN comprenant une séquence de nucléotide qui code pour un activateur de plasminogène modifié suivant l'une quelconque des revendications 1 à 11.

20. Vecteur d'expression réplicable capable, dans une cellule hôte, d'exprimer un polymère d'ADN suivant la revendication 19.

21. Cellule hôte transformée avec le vecteur suivant la revendication 20.

22. Chaîne d'activateur de plasminogène qui a été modifié suivant l'une quelconque des revendications 1 à 11.

**23.** Polymère d'ADN comprenant une séquence de nucléotide qui code pour la chaîne d'activateur de plasminogène modifié suivant la revendication 22.

**24.** Protéine hybride comprenant la chaîne modifiée suivant la revendication 22 liée à une chaîne d'une autre protéase active fibrinolytiquement, de telle sorte que la protéine hybride a un site catalytique essentiel pour l'activité fibrinolytique qui est éventuellement bloquée par un groupe bloquant pouvant être éliminé.

**25.** Composition pharmaceutique comprenant un activateur de plasminogène modifié suivant l'une quelconque des revendications 1 à 11, un dérivé ou un conjugué suivant l'une quelconque des revendications 12 à 18 ou une protéine hybride suivant la revendication 24, en combinaison avec un support acceptable du point de vue pharmaceutique.

**26.** Activateur de plasminogène modifié suivant l'une quelconque des revendications 1 à 11, un dérivé ou conjugué suivant l'une quelconque des revendications 12 à 18 ou protéine hybride suivant la revendication 24 , utile en tant que substance thérapeutique active.

**27.** Activateur de plasminogène modifié suivant l'une quelconque des revendications 1 à 11, un dérivé ou un conjugué suivant l'une quelconque des revendications 12 à 18 ou protéine hybride suivant la revendication 24, utile dans le traitement d'une maladie thrombotique.

**28.** Utilisation d'un activateur de plasminogène modifié suivant l'une quelconque des revendications 1 à 11, d'un dérivé ou d'un conjugué suivant l'une quelconque des revendications 12 à 18 ou d'une protéine hybride suivant la revendication 24 pour la préparation d'un médicament pour le traitement d'une maladie thrombotique.

**29.** Procédé pour préparer un activateur de plasminogène modifié suivant la revendication 1 ou un dérivé suivant la revendication 16, lequel procédé comprend l'expression d'un ADN codant pour cet activateur de plasminogène modifié dans une cellule hôte recombinante et la récupération de l'activateur de plasminogène modifié produit et ensuite, le blocage éventuel d'un quelconque site catalytique avec un groupe bloquant pouvant être éliminé.

**30.** Procédé pour préparer un polymère d'ADN suivant la revendication 19, par la condensation des unités nucléotidiques appropriées mono-, di- ou oligomère.

**31.** Activateur de plasminogène modifié pouvant être obtenu par le procédé suivant la revendication 29.

**Revendications pour l'Etat contractant suivant : ES**

**1.** Procédé pour préparer un activateur de plasminogène actif fibrinolytiquement qui a été modifié par l'addition d'un ou de plusieurs domaines de doigt et dans lequel un quelconque site catalytique responsable de l'activité fibrinolytique est éventuellement bloqué par un groupe bloquant pouvant être éliminé, lequel procédé comprend l'expression d'un ADN codant pour cet activateur de plasminogène modifié dans une cellule hôte recombinante et la récupération de l'activateur de plasminogène modifié produit et ensuite, le blocage éventuel d'un quelconque site catalytique avec un groupe bloquant pouvant être éliminé.

**2.** Procédé suivant la revendication 1, dans lequel le ou chaque domaine de doigt provient de t-PA naturel.

**3.** Procédé suivant la revendication 2, dans lequel la séquence d'acide aminé du ou de chaque domaine de doigt se prolonge au N-terminal pour comprendre les résidus 1 à 5 de t-PA naturel.

**4.** Procédé suivant l'une quelconque des revendications précédentes, dans lequel au moins un des domaines du doigt est lié à une seconde séquence d'acides aminés qui correspond au domaine de facteur de croissance du t-PA naturel.

**5.** Procédé suivant l'une quelconque des revendications précédentes, dans lequel la modification est au N-terminal de l'activateur de plasminogène.

**6.** Procédé suivant la revendication 1 pour préparer un activateur de plasminogène modifié qui comprend une ou plusieurs unités N-terminal de la forme A-F-X et/ou A-F-Y-G-Z-,dans lesquelles F représente les résidus 6 à 43 de t-PA, G représente les résidus 51 à 84 de t-PA, X, Y et Z représentent des liaisons ou des séquences de liaison d'acides aminés qui peuvent être introduits par synthèse pendant la préparation de l'activateur de plasminogène modifié et/ou provenir des séquences de t-PA naturel adjacentes aux domaines du doigt et de facteur de croissance et A est un prolongement N-terminal éventuel du domaine du doigt.

**7.** Procédé suivant la revendication 6, dans lequel la séquence de liaison X ou Z se termine avec un résidu autre que l'arginine ou la lysine.

**8.** Procédé suivant la revendication 7, dans lequel X comprend les résidus 44 à 50 et 88 à 91 du t-PA naturel liés au C-terminal à la séquence -pro-gly-, Y comprend les résidus 44 à 50 du t-PA naturel, Z comprend les résidus 85 à 91 du t-PA naturel liés au C-terminal à la séquence -pro-gly- et A comprend les résidus 1 à 5 du t-PA naturel.

**9.** Procédé suivant l'une quelconque des revendications précédentes pour préparer un activateur de plasminogène modifié qui comprend 1 à 6 domaines de doigt supplémentaires.

**10.** Procédé suivant l'une quelconque des revendications précédentes, dans lequel l'activateur de plasminogène est le t-PA naturel, le t-PA modifié dans la région du domaine de facteur de croissance ou le t-PA comprenant un acide aminé sans chaîne latérale chargée à la place de la lysine en position 277.

**11.** Procédé suivant la revendication 1, pour préparer un activateur de plasminogène modifié choisi parmi les suivants :

$\lceil$GARSYQVI$\rfloor$F$\lceil$HSVPVKS$\rfloor$G$\lceil$EIDTRATPG$\rfloor$-t-PA

$\lceil$GARSYQVI$\rfloor$F$\lceil$HSVPVKSTRATPG$\rfloor$-t-PA

$\lceil$GARSYQVI$\rfloor$F$\lceil$HSVPVKS$\rfloor$G$\lceil$EIDTRATPG$\rfloor$-t-PA(lys$_{277}$->ile$_{277}$)

$\lceil$GARSYQVI$\rfloor$F$\lceil$HSVPVKSTRAT(PGSG)$_n$PG$\rfloor$-t-PA(lys$_{277}$->ile$_{277}$)

$\lceil$GARSYQVI$\rfloor$F$\lceil$HSVPVKS$\rfloor$G$\lceil$EIDTRATPG$\rfloor$-t-PA(des cys$_{51}$ à asp$_{87}$)

$\lceil$GARSYQVI$\rfloor$F$\lceil$HSVPVKSTRATPH$\rfloor$-t-PA(des cys$_{51}$ à asp$_{87}$)

dans lesquels t-PA représente le t-PA naturel avec un résidu sérine N-terminal, F représente les résidus 6 à 43 du t-PA naturel, G représente les résidus 51 à 84 du t-PA naturel, n est un entier d'au moins 1 et les symboles entre crochets représentent les résidus d'acides aminés selon la notation des acides aminés par une lettre unique, ou une variante de ceux-ci issue du traitement du N-terminal.

**12.** Procédé pour préparer un polymère d'ADN comprenant une séquence de nucléotide qui code pour un activateur de plasminogène modifié tel que défini dans l'une quelconque des revendications 1 à 11, par la condensation des unités de nucléotide appropriées mono-,di- ou oligomères.

**13.** Procédé pour préparer un vecteur d'expression réplicable capable, dans une cellule hôte, d'exprimer un polymère d'ADN tel que défini dans la revendication 12, lequel procédé comprend la coupure d'un vecteur compatible avec cette cellule hôte pour fournir un segment d'ADN linéaire ayant un réplicon intact, et la combinaison de ce segment linéaire avec une ou plusieurs molécules d'ADN qui, avec ce segment linéaire,codent pour l'activateur de plasminogène modifié dans des conditions de ligature.

**14.** Procédé pour préparer une cellule hôte transformée avec le vecteur défini dans la revendication 13, lequel procédé comprend la transformation d'une cellule hôte avec ce vecteur dans des conditions de transformation.

**15.** Procédé pour préparer une chaîne d'activateur de plasminogène qui a été modifiée suivant l'une quelconque des revendications 1 à 11, lequel procédé comprend la séparation de cette chaîne de l'autre chaîne de celui-ci par une réduction douce ou l'expression d'un ADN codant pour celle-ci, dans une cellule hôte recombinante et la récupération de la chaîne modifiée produite.

**16.** Procédé pour préparer une protéine hybride comprenant la chaîne modifiée définie dans la revendication 15 liée à une autre chaîne d'une autre protéase active fibrinolytiquement, de telle sorte que la protéine hybride a un site catalytique essentiel pour l'activité fibrinolytique qui est éventuellement bloqué par un groupe bloquant pouvant être éliminé, lequel procédé comprend le mélange de ces chaînes dans des conditions oxydantes ou la ligature de l'ADN codant pour ces chaînes et l'expression de l'ADN soudé dans une cellule procaryotique ou eucaryotique, et ensuite le blocage éventuel du site catalytique de la protéine hybride produite avec un groupe bloquant pouvant être éliminé.

**17.** Utilisation d'un activateur de plasminogène modifié suivant l'une quelconque des revendications 1 à 11 pour la préparation d'un médicament pour le traitement de maladies thrombotiques.

**18.** Activateur de plasminogène actif fibrinolytiquement pouvant être obtenu par le procédé suivant la revendication 1 .

**Patentansprüche**
**Patentansprüche für folgende Vertragsstaaten : BE, CH, DE, FR, GB, GR, IT, LU, NL, SE**

**1.** Fibrinolytisch aktiver Plasminogen-Aktivator, der durch das Hinzufügen von einer oder mehreren Fingerdomänen modifiziert worden ist.

**2.** Modifizierter Plasminogen-Aktivator nach Anspruch 1, wobei die oder jede Fingerdomäne von nativem t-PA stammt.

**3.** Modifizierter Plasminogen-Aktivator nach Anspruch 2, wobei die Aminosäuresequenz der oder jeder Fingerdomäne am N-Terminus verlängert ist, um die Reste 1 bis 5 von nativem t-PA zu umfassen.

**4.** Modifizierter Plasminogen-Aktivator nach einem der vorstehenden Ansprüche, wobei mindestens eine der Fingerdomänen mit einer zweiten Aminosäuresequenz verbunden ist, die der Wachstumsfaktordomäne von nativem t-PA entspricht.

**5.** Modifizierter Plasminogen-Aktivator nach einem der vorstehenden Ansprüche, wobei sich die Modifikation am N-Terminus des Plasminogen-Aktivators befindet.

**6.** Modifizierter Plasminogen-Aktivator nach Anspruch 1, der eine oder mehrere N-terminale Einheiten der Form A-F-X und/oder A-F-Y-G-Z- umfaßt, wobei F die Reste 6 bis 43 von t-PA, G die Reste 51 bis 84 von t-PA, X, Y und Z Bindungen oder Verbindungssequenzen von Aminosäuren darstellen, die während der Herstellung des modifizierten Plasminogen-Aktivators synthetisch eingeführt werden können und/oder von den an die Finger- und Wachstumsfaktordomänen angrenzenden nativen t-PA-Sequenzen stammen können und A eine mögliche N-terminale Verlängerung der Fingerdomäne darstellt.

**7.** Modifizierter Plasminogen-Aktivator nach Anspruch 6, wobei die Verbindungssequenz X oder Z mit einem anderen Rest als Arginin oder Lysin endet.

**8.** Modifizierter Plasminogen-Aktivator nach Anspruch 7, wobei X die Reste 44 bis 50 und 88 bis 91 von nativem t-PA, verbunden am C-Terminus mit der Sequenz -Pro-Gly-, Y die Reste 44 bis 50 von nativem t-PA, Z die Reste 85 bis 91 von nativem t-PA, verbunden am C-Terminus mit der Sequenz -Pro-Gly-, und A die Reste 1 bis 5 von nativem t-PA umfaßt.

**9.** Modifizierter Plasminogen-Aktivator nach einem der vorstehenden Ansprüche, der 1 bis 6 zusätzliche Fingerdomänen umfaßt.

**10.** Modifizierter Plasminogen-Aktivator nach einem der vorstehenden Ansprüche, wobei der Plasminogen-Aktivator ein nativer t-PA, im Bereich der Wachstumsfaktordomäne modifizierter t-PA oder t-PA ist, der anstelle von Lysin in Position 277 eine Aminosäure ohne eine geladene Seitenkette enthält.

11. Modifizierter Plasminogen-Aktivator ausgewählt aus

$$[GARSYQVI]F[HSVPVKS]G[EIDTRATPG]-t-PA$$
$$[GARSYQVI]F[HSVPVKSTRATPG]-t-PA$$
$$[GARSYQVI]F[HSVPVKS]G[EIDTRATPG]-t-PA(lys_{277}->ile_{277})$$
$$[GARSYQVI]F[HSVPVKSTRAT(PGSG)_nPG]-t-PA(lys_{277}->ile_{277})$$
$$[GARSYQVI]F[HSVPVKS]G[EIDTRATPG]-t-PA(des\ cys_{51}\ to\ asp_{87})$$
$$[GARSYQVI]F[HSVPVKSTRATPG]-t-PA(des\ cys_{51}\ to\ asp_{87})$$

wobei t-PA nativen t-PA mit einem N-terminalen Serinrest, F die Reste 6 bis 43 von nativem t-PA, G die Reste 51 bis 84 von nativem t-PA darstellt, n eine ganze Zahl vom Mindestwert 1 ist und die Symbole in eckigen Klammern Aminosäurereste nach der Ein-Buchstaben-Schreibweise für Aminosäuren oder eine am N-Terminus prozessierte Variante davon darstellen.

12. Fibrinolytisches aktives Enzym-Protein-Konjugat, in dem die für die fibrinolytische Aktivität verantwortliche katalytische Stelle auf dem Enzym durch ein über eine reversible Verbindungsgruppe daran angeheftetes menschliches Protein blockiert ist, wobei das Enzym und/oder das menschliche Protein der modifizierte Plasminogen-Aktivator nach einem der Ansprüche 1 bis 11 ist.

13. Fibrinolytisch aktives Enzym-Protein-Konjugat, enthaltend mindestens ein fibrinolytisches Enzym, das über eine andere Stelle als die für die fibrinolytische Aktivität verantwortliche katalytische Stelle an mindestens ein menschliches Protein gebunden ist, wobei das Enzym und/oder das menschliche Protein der modifizierte Plasminogen-Aktivator nach einem der Ansprüche 1 bis 11 ist.

14. Konjugat des modifizierten Plasminogen-Aktivators nach einem der Ansprüche 1 bis 11, wobei der modifizierte Plasminogen-Aktivator durch eine reversible Verbindungsgruppe mit mindestens einem wasserlöslichen Polymer verbunden ist.

15. Enzym-Konjugat enthaltend eine Vielzahl von fibrinolytischen Enzymen, die über ihre aktiven Zentren durch eine entfernbare Blockierungsgruppe miteinander verbunden sind, wobei mindestens eines der Enzyme der modifizierte Plasminogen-Aktivator nach einem der Ansprüche 1 bis 11 ist.

16. Derivat des modifizierten Plasminogen-Aktivators nach einem der Ansprüche 1 bis 11 oder Konjugat nach einem der Ansprüche 12 bis 14, bei dem jede für die fibrinolytische Aktivität verantwortliche katalytische Stelle durch eine entfernbare Blockierungsgruppe blockiert ist.

17. Derivat nach Anspruch 16, wobei die entfernbare Blockierungsgruppe gegebenenfalls substituiertes Benzoyl oder Acryloyl ist.

18. Derivat nach Anspruch 17, wobei die möglichen Substituenten für die Benzoylblockierungsgruppe Halogen, $C_{1-6}$-Alkyl, $C_{1-6}$-Alkoxy, $C_{1-6}$-Alkanoyloxy, $C_{1-6}$-Alkanoylamino, Amino und Guanidino einschließen.

19. DNA-Polymer, umfassend eine Nukleotidsequenz, die einen modifizierten Plasminogen-Aktivator nach einem der Ansprüche 1 bis 11 codiert.

20. Replizierbarer Expressionsvektor, der in einer Wirtszelle zur Expression eines DNA-Polymers nach Anspruch 19 fähig ist.

21. Wirtszelle, transformiert mit einem Vektor nach Anspruch 20.

22. Plasminogen-Aktivator-Kette, die gemäß einem der Ansprüche 1 bis 11 modifiziert ist.

**23.** DNA-Polymer, umfassend eine Nukleotidsequenz, die die modifizierte Plasminogen-Aktivator-Kette nach Anspruch 22 codiert.

**24.** Hybridprotein, umfassend die modifizierte Kette nach Anspruch 22, verbunden mit einer Kette einer anderen fibrinolytisch aktiven Protease, so daß das Hybridprotein eine für die fibrinolytische Aktivität wesentliche katalytische Stelle aufweist, die gegebenenfalls durch eine entfernbare Blockierungsgruppe blockiert ist.

**25.** Arzneimittel, enthaltend modifizierten Plasminogen-Aktivator nach einem der Ansprüche 1 bis 11, ein Derivat oder Konjugat nach einem der Ansprüche 12 bis 18 oder ein Hybridprotein nach Anspruch 24 in Kombination mit einem pharmazeutisch verträglichen Träger.

**26.** Modifizierter Plasminogen-Aktivator nach einem der Ansprüche 1 bis 11, ein Derivat oder Konjugat nach einem der Ansprüche 12 bis 18 oder ein Hybridprotein nach Anspruch 24 zur Verwendung als therapeutisch aktiver Stoff.

**27.** Modifizierter Plasminogen-Aktivator nach einem der Ansprüche 1 bis 11, ein Derivat oder Konjugat nach einem der Ansprüche 12 bis 18 oder ein Hybridprotein nach Anspruch 24 zur Verwendung bei der Behandlung einer thrombotischen Erkrankung.

**28.** Verwendung eines modifizierten Plasminogen-Aktivators nach einem der Ansprüche 1 bis 11, eines Derivates oder Konjugates nach einem der Ansprüche 12 bis 18 oder eines Hybridproteins nach Anspruch 24 zur Herstellung eines Arzneimittels für die Behandlung einer thrombotischen Erkrankung.

**29.** Verfahren zur Herstellung von modifiziertem Plasminogen-Aktivator nach Anspruch 1 oder einem Derivat nach Anspruch 16, umfassend die Expression der den modifizierten Plasminogen-Aktivator codierenden DNA in einer rekombinanten Wirtszelle und Gewinnung des modifizierten Plasminogen-Aktivator-Produkts und nachfolgend gegebenenfalls Blockierung jeder katalytischen Stelle mit einer entfernbaren Blockierungsgruppe.

**30.** Verfahren zur Herstellung eines DNA-Polymers nach Anspruch 14 durch Kondensation von geeigneten mono-, di- oder oligomeren Nukleotideinheiten.

**31.** Modifizierter Plasminogen-Aktivator erhältlich durch das Verfahren von Anspruch 29.

**Patentansprüche für folgenden Vertragsstaat : ES**

**1.** Verfahren zur Herstellung eines fibrinolytisch aktiven Plasminogen-Aktivators, der durch das Hinzufügen von einer oder mehreren Fingerdomänen modifiziert worden ist und in dem jede für die fibrinolytische Aktivität verantwortliche katalytische Stelle gegebenenfalls durch eine entfernbare Blockierungsgruppe blockiert ist, umfassend die Expression der den modifizierten Plasminogen-Aktivator codierenden DNA in einer rekombinanten Wirtszelle und Gewinnung des modifizierten Plasminogen-Aktivator-Produkts und nachfolgend gegebenenfalls Blockierung jeder katalytischen Stelle mit einer entfernbaren Blockierungsgruppe.

**2.** Verfahren nach Anspruch 1, wobei die oder jede Fingerdomäne von nativem t-PA stammt.

**3.** Verfahren nach Anspruch 2, wobei die Aminosäuresequenz der oder jeder Fingerdomäne am N-Terminus verlängert ist, um die Reste 1 bis 5 von nativem t-PA zu umfassen.

**4.** Verfahren nach einem der vorstehenden Ansprüche, wobei mindestens eine der Fingerdomänen mit einer zweiten Aminosäuresequenz verbunden ist, die der Wachstumsfaktordomäne von nativem t-PA entspricht.

**5.** Verfahren nach einem der vorstehenden Ansprüche, wobei sich die Modifikation am N-Terminus des Plasminogen-Aktivators befindet.

**6.** Verfahren nach Anspruch 1 zur Herstellung eines modifizierten Plasminogen-Aktivators, der eine oder mehrere N-terminale Einheiten der Form A-F-X und/oder A-F-Y-G-Z-umfaßt, wobei F die Reste 6 bis 43 von t-PA, G die Reste 51 bis 84 von t-PA, X, Y und Z Bindungen oder Verbindungssequenzen von Aminosäuren darstellen, die während der Herstellung des modifizierten Plasminogen-Aktivators synthetisch eingeführt werden können und/oder von den an die Finger- und Wachstumsfaktordomänen angrenzenden nativen t-PA-Sequenzen stammen können und A eine mögliche N-terminale Verlängerung der Fingerdomäne darstellt.

7. Verfahren nach Anspruch 6, wobei die Verbindungssequenz X oder Z mit einem anderen Rest als Arginin oder Lysin endet.

8. Verfahren nach Anspruch 7, wobei X die Reste 44 bis 50 und 88 bis 91 von nativem t-PA, verbunden am C-Terminus mit der Sequenz -Pro-Gly-, Y die Reste 44 bis 50 von nativem t-PA, Z die Reste 85 bis 91 von nativem t-PA, verbunden am C-Terminus mit der Sequenz -Pro-Gly-, und A die Reste 1 bis 5 von nativem t-PA umfaßt.

9. Verfahren nach einem der vorstehenden Ansprüche zur Herstellung eines modifizierten Plasminogen-Aktivators, der 1 bis 6 zusätzliche Fingerdomänen umfaßt.

10. Verfahren nach einem der vorstehenden Ansprüche, wobei der Plasminogen-Aktivator nativer t-PA, im Bereich der Wachstumsfaktordomäne modifizierter t-PA oder t-PA ist, der anstelle von Lysin in Position 277 eine Aminosäure ohne eine geladene Seitenkette enthält.

11. Verfahren nach Anspruch 1 zur Herstellung eines modifizierten Plasminogen-Aktivators ausgewählt aus

$$[GARSYQVI]F[HSVPVKS]G[EIDTRATPG]-t-PA$$

$$[GARSYQVI]F[HSVPVKSTRATPG]-t-PA$$

$$[GARSYQVI]F[HSVPVKS]G[EIDTRATPG]-t-PA(lys_{277}->ile_{277})$$

$$[GARSYQVI]F[HSVPVKSTRAT(PGSG)_{n}PG]-t-PA(lys_{277}->ile_{277})$$

$$[GARSYQVI]F[HSVPVKS]G[EIDTRATPG]-t-PA(des\ cys_{51}\ to\ asp_{87})$$

$$[GARSYQVI]F[HSVPVKSTRATPG]-t-PA(des\ cys_{51}\ to\ asp_{87})$$

wobei t-PA nativer t-PA mit einem N-terminalen Serinrest, F die Reste 6 bis 43 von nativem t-PA, G die Reste 51 bis 84 von nativem t-PA darstellt, n eine ganze Zahl vom Mindestwert 1 ist und die Symbole in eckigen Klammern Aminosäurereste nach der Ein-Buchstaben-Schreibweise für Aminosäuren oder eine am N-Terminus prozessierte Variante davon darstellt.

12. Verfahren zur Herstellung eines DNA-Polymers, umfassend eine Nukleotidsequenz, die einen modifizierten Plasminogen-Aktivator, wie in einem der Ansprüche 1 bis 11 definiert, codiert, durch Kondensation von geeigneten mono-, di- oder oligomeren Nukleotideinheiten.

13. Verfahren zur Herstellung eines replizierbaren Expressionsvektors, der in einer Wirtszelle zur Expression eines wie in Anspruch 12 definierten DNA-Polymers fähig ist, umfassend die Spaltung eines mit der Wirtszelle kompatiblen Vektors, um ein ein intaktes Replikon aufweisendes lineares DNA-Segment bereitzustellen, und die Kombination des linearen Segments mit einem oder mehreren DNA-Molekülen, die unter Ligierungsbedingungen zusammen mit dem linearen Segment den modifizierten Plasminogen-Aktivator codieren.

14. Verfahren zur Herstellung einer mit dem in Anspruch 13 definierten Vektor transformierten Wirtszelle, umfassend die Transformation einer Wirtszelle mit dem Vektor unter Transformationsbedingungen.

15. Verfahren zur Herstellung einer Plasminogen-Aktivator-Kette, die nach einem der Ansprüche 1 bis 11 modifiziert worden ist, umfassend die Abtrennung der Kette von der anderen Kette durch milde Reduktion oder die Expression dafür codierender DNA in einer rekombinanten Wirtszelle und Gewinnung des modifizierten Kettenprodukts.

**16.** Verfahren zur Herstellung von Hybridprotein enthaltend die in Anspruch 15 definierte modifizierte Kette verbunden mit einer Kette einer anderen fibrinolytisch aktiven Protease, so daß das Hybridprotein eine für die fibrinolytische Aktivität wesentliche katalytische Stelle aufweist, die gegebenenfalls durch eine entfernbare Blockierungsgruppe blockiert ist, umfassend das Mischen der Kette unter oxidativen Bedingungen oder Ligierung von die Ketten codierender DNA und Expression der ligierten DNA in einem prokaryontischen oder eukaryontischen Wirt und nachfolgend gegebenenfalls Blockierung der katalytischen Stelle des Hybridproteinproduktes mit einer entfernbaren Blockierungsgruppe.

**17.** Verwendung eines wie in einem der Ansprüche 1 bis 11 definierten modifizierten Plasminogen-Aktivators zur Herstellung eines Arzneimittels für die Behandlung einer thrombotischen Erkrankung.

**18.** Fibrinolytisch aktiver Plasminogen-Aktivator erhältlich durch das Verfahren nach Anspruch 1.